(19) Europäisches Patentamt
European Patent Office
Office européen des brevets

(11) Publication number: 0 193 227
B1

(12) # EUROPEAN PATENT SPECIFICATION

(45) Date of publication of the patent specification: 11.04.90

(51) Int. Cl.⁴: C07D 303/22, C07D 303/24, C07D 301/02, C07C 217/30, C12P 17/02

(21) Application number: 86200198.9

(22) Date of filing: 11.02.86

(54) **Process for the preparation of arylglycidyl ethers and 3-substituted 1-alkylamino-2-propanols.**

(30) Priority: 16.10.85 GB 8525456
13.02.85 GB 8503665

(43) Date of publication of application:
03.09.86 Bulletin 86/36

(45) Publication of the grant of the patent:
11.04.90 Bulletin 90/15

(84) Designated Contracting States:
AT BE CH DE FR GB IT LI LU NL SE

(56) References cited:
EP-A-16 652 7
FR-A- 2 250 752

AGRICULTURAL & BIOLOGICAL CHEMISTRY, vol. 43, no. 10, October 1979, pages 2099-2104, Tokyo, JP; H. OHTA et al.: "Asymmetric epoxidation of long chain terminal olefins by Corynebacterium equi IFO 3730"
JOURNAL OF ORGANIC CHEMISTRY, vol. 46, no. 15, 17th July 1981, pages 3128-3131, American Chemical Society, Columbus, Ohio, US; M.-J. DE SMET et al.: "Practical approach to high-yield enzymatic stereospecific organic synthesis in multiphase systems" 000

The file contains technical information submitted after

(73) Proprietor: GIST-BROCADES N.V., Wateringseweg 1, NL-2611 XT Delft(NL)
Proprietor: SHELL INTERNATIONALE RESEARCH MAATSCHAPPIJ B.V., Carel van Bylandtlaan 30, NL-2596 HR Den Haag(NL)

(72) Inventor: Phillips, Gareth Thomas, 5 The Finches, Sittingbourne Kent(GB)
Inventor: Robertson, Brian William, 1616 Lords Close Bapchild, Sittingbourne Kent ME9 8AG(GB)
Inventor: Bertola, Mauro Attilio, A. van Scheltemaplein 91, NL-2624 PJ Delft(NL)
Inventor: Koger, Hein Simon, G. Vermootenstraat 32, NL-2064 XR Spaarndam(NL)
Inventor: Marx, Arthur Friedrich, Florence Nightingalelaan 12, NL-2614 GM Delft(NL)
Inventor: Watts, Peter Douglas, 9 Woollett Road, Sittingbourne Kent(GB)

(74) Representative: Huygens, Arthur Victor, Dr. et al, c/o Gist-Brocades N.V. Patent & Trademarks Department Wateringseweg 1 PO Box 1, NL-2600 MA Delft(NL)

(56) References cited: (continuation)
the application was filed and not included in this specification

EP 0 193 227 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

**Description**

The present invention relates to a process for the preparation of a pharmaceutically active compound in an stereospecific form of the formula

$$R_1–O–CH_2–CHOH–CH_2–NH–R_2 \ (I)$$

or a pharmaceutically acceptable salt thereof, like an acid addition salt, and/or a compound in a stereospecific form of the formula

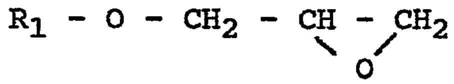

(II)

wherein $R_1$ is an optionally substituted phenyl group and wherein $R_2$ is an alkyl group of 2 to 6 carbon atoms, this alkyl group being optionally substituted, which comprises subjecting a compound of the formula

$$R_1–O–CH_2–CH{=}CH_2 \ (III)$$

to the action of a bacterium belonging to the genus Rhodococcus, the genus Mycobacterium, the genus Nocardia or the genus Pseudomonas, having the ability in a suitable medium for stereoselective epoxidation of compound (III) into compound (II), whereby at least 90% by weight of compound (II) is formed in the S configuration, at least partly separating compound (II) and/or reacting compound (II) with a $C_2–C_6$ alkylamine the alkyl group of which is optionally substituted and at least partly separating compound (I) and/or converting compound (I) into the pharmaceutically acceptable salt thereof.

It is commonly known that a lot of biologically active compounds exist as a mixture of stereoisomers. Most frequently these mixtures are used as such in agricultural and pharmaceutical applications. The major reason is that the separation costs still exceed the potential advantage of increase in activity. Usually the required biological activity resides in one stereoisomer so that at best the potency of the mixture is reduced to half. However it is apparent that modern pharmacologists are becoming increasingly aware of other implications of administering mixtures wherein one or more stereoisomers are regarded as an impurity that may not have the desired therapeutic effect but may well have other unwanted physiological effects including toxicity. Some examples are cited to illustrate the association of biological activity with single stereoisomers.

Within the pharmaceutical area most of the beta-adrenergic blocking agents are sold as mixtures although the activity resides in one stereoisomer. In one instance a drug known as labetalol has a combined alpha-adrenergic blocking and beta-adrenergic blocking action that has been shown to be attributed to two separate pairs of isomers from the mixture of four. N. Toda et al. reported in J.Pharmacol.Exp.Ther. 207 (1978) 311 that (-)-metoprolol is 270 to 380 times more potent than (+)-metoprolol in attenuating the response of rabbit atria and tracheal muscles to isoproterenol (a beta-adrenoreceptor stimulant).

Current routes to single stereoisomer beta-blockers generally involve chemical resolutions or rather lengthy chemical syntheses from stereomeric precursors which are for example described in US patent 4,408,063 and in J.Org.Chem. 41 (1976) 3121 by L.M. Weinstok et al.. Thus, these described processes to prepare analogously the S enantiomer (optically active stereoisomer) of metoprolol are not economically advantageous in industrial applications. Therefore an object of the present invention is to provide an efficient process for the preparation of such stereoisomers which may be carried out on an industrial scale in an economically attractive way.

The ability of microorganisms to convert stereospecifically short-chain alkenes ($C_1–C_4$) into their corresponding epoxyalkanes in a gas/solid or in a two-liquid phase bioreactor is demonstrated by J. Tramper et al. (3rd European Congress on Biotechnology, München, 10–14 September 1984). Another example of the microbiological preparation of epoxyalkanes is disclosed in the U.S. patent 4 106 986 describing the conversion of straight-chain 1-alkenes ($C_1–C_{20}$) into 1,2-epoxyalkanes. In the European patent application 0099609 examples are given of the conversion of propene and 1-octene into their corresponding epoxyalkanes. However, the conversion of substituted alkenes, such as an ether compound (III), can in no way be deduced from these known processes, which are only applicable to straight or sometimes branches alkenes.

EP-A-166 527 relates to the preparation of 2,3-epoxypropylethers from the corresponding allylethers by means of epoxide producing microorganisms belonging to the genera Nocardia, Brevibacterium, Corynebacterium, Pseudomonas, Rhodococcus, Arthrobacter and Micrococcus. However, according to this application 79% enantiomeric excess in the S configuration is obtained at highest, while most epoxidations resulted in lower optical purities or even in epoxides which have mainly the R configuration.

EP-A-166 527, designating CH, DE, FR, GB, IT, LI, NL and SE is falling within the terms of Article 54, paragraph 3 of the European Patent Convention and for these designating countries does form part of the state of the art concerning novelty but is not relevant to the question of inventive step.

As a result of extensive research and experimentation an improved synthesis has now surprisingly been found for the preparation of particularly the S enantiomer of compound (I) and the S enantiomer of compound (II), starting from a compound (III), using bacteria being active for the epoxidation of compound (III) to produce compound (II) which is showing at least 80% by weight the S configuration, whereafter at least partly compound (II) is separated and/or said compound (II) is reacted with an alkylamine to produce said compound (I).

More specifically the present invention relates to a process for the preparation of a pharmaceutically active compound in a stereospecific form of the formula (I) or a pharmaceutically acceptable salt thereof, like an acid addition salt, and/or compound in a stereospecific form of formula (II), wherein $R_1$ is an optionally substituted phenyl group and wherein $R_2$ is an alkyl group of 2 to 6 carbon atoms, this alkyl group being optionally substituted, which comprises subjecting a compound of the formula (III) to the action of a microorganism having the ability for stereoselective epoxidation of compound (III) into compound (II), having at least 80% by weight the S configuration, at least partly separating compound (II) and/or reacting compound (II) with a $C_2$–$C_6$ alkylamine the alkyl group of which is optionally substituted and at least partly separating compound (I) and/or converting compound (I) into the pharmaceutically acceptable salt thereof. Preferably the alkylamine is isopropylamine or tertiary butylamine.

More preferably $R_1$ is a 4-acetamidophenyl, 4-carbamoylmethylphenyl, 4-[2-(methylformylamino)-ethyl]-phenyl, phenyl, 4-(3-cyclohexylureido)-phenyl, 4-(2-methoxyethoxy)-phenyl or 4-[2-(cyclopropylmethoxy)-ethyl]-phenyl group.

Preferably the processes of the present invention are carried out, starting with a compound (III) wherein $R_1$ represents carbamoylmethylphenyl or phenyl. In a preferred embodiment, the process is carried out in such a way by selecting proper microorganisms that at least 90% by weight of the S configuration is formed.

Cross reference is made to the copending European application EP-A-193 228 in which especially the epoxidation of 4-(2-methoxyethyl)-phenylallyl ether by bacteria is described.

With the term suitable microorganisms is meant for example bacteria belonging to the genera Rhodococcus, Mycobacterium, Nocardia and Pseudomonas. The micro-organisms are optionally immobilized with polymer gel. The micro-organisms for the epoxidation of 4-(2-methoxyethyl)-phenylallyl ether include cultures of species Nocardia corallina (an example of this species is deposited in the ATCC under the accession number of 31338), species Rhodococcus sp. (an example of this species is deposited in the NCIB under the accession number of 11277), species Mycobacterium rhodochrous (an example of this species is deposited in the NCIB under the accession number of 9703), species Rhodococcus equi (an example of this species is deposited in the NCIB under the accession number of 12035), species Pseudomonas aeruginosa (an example of this species is deposited in the NCIB under the accession number 12036), species Pseudomonas oleovorans (an example of this species is deposited in the ATCC under the accession number of 29347), species Pseudomonas putida (an example of this species is deposited in the NCIB under the accession number of 9571) and species Pseudomonas aeruginosa (an example of this species is deposited in the NCIB under the accession number of 8704).

The micro-organisms for the epoxidation of phenylallyl ether include a culture of the species Mycobacterium WMI (an example of this species is deposited in the NCIB under the accession number of 11626).

The micro-organisms for the epoxidation of 4-allyloxy-phenylacetamide include a culture of the species Pseudomonas oleovorans (an example of this species is deposited in the ATCC under the accession number of 29347).

In practising the preferred embodiment of the process of the present invention, a micro-organism having the ability to convert 4-(2-methoxyethyl)-phenylallyl ether into 4-(2-methoxyethyl)-phenylglycidyl ether having at least 90% by weight the S configuration, has to be selected from the above-mentioned microorganisms, to be cultured for 0.5 to 10 days, whereafter the bacterial cells have to be collected from the culture solution, the cells are suspended in a liquid nutrient medium and 4-(2-methoxyethyl)-phenylallyl ether is subjected to the action of the cells.

The microorganisms used in the present invention showing the epoxidation activity, have to be cultured for about 0.5 to 10 days, whereafter the cells are suspended in a liquid nutrient medium, preferably a minimal liquid nutrient medium, and compound (III) is subjected to the action of the cells. After the abovementioned cultivation for about 0.5 to 10 days the cells may be isolated from the culturing medium before suspending the cells in the minimal liquid nutrient medium. To grow the micro-organisms used for the stereo selective epoxidation of compound (III), ordinary culture mediums containing an assimilable carbon source (for example glucose, lactate, hydrocarbons like tetradecane ($C_{14}$), etc.), a nitrogen source (for example ammonium sulphate, ammonium nitrate, ammonium chloride, etc.), with an agent for an organic nutrient source (for example yeast extract, malt extract, peptone, meat extract, etc.) and an inorganic nutrient source (for example phosphate, magnesium, potassium, zinc, iron and other metals in trace amounts) may be used. Optionally an inducer (for example diethoxymethane) is added to the culture medium. A temperature between 0 and 45°C and a pH between 3.5 and 9 is maintained during the growth of the micro-organisms. Preferably the micro-organisms are grown at a temperature between 20 and 37°C and at a pH between 5 and 8.

The aerobic conditions required during the growth of the micro-organisms can be provided according

to any of the well-established procedures, provided that the supply of oxygen is sufficient to meet the metabolic requirement of the micro-organisms. This is most conveniently achieved by supplying a gaseous oxygen, preferably in the form of air. During the conversion of compound (III) into compound (II) the micro-organisms might be in a growing stage using an abovementioned ordinary culture medium. The microorganisms may be supplemented with a cosubstrate.

Preferably during the conversion of compound (III) into compound (II), the micro-organisms can be held in a substantially non-growing stage using a minimal culture medium. As minimal culture medium, an ordinary culture medium may be used containing an assimilable carbon source when required (for example glucose, lactate, hydrocarbons like tetradecane ($C_{14}$), etc.), a nitrogen source when required (for example ammonium sulphate, ammonium nitrate, ammonium chloride, etc.), with an agent for an organic nutrient source when required (for example yeast extract, malt extract, peptone, meat extract etc.) and an inorganic nutrient source when required (for example phosphate, magnesium, potassium, zinc, iron and other metals in trace amounts). The micro-organisms can be kept in the non-growing stage for example under exclusion of the assimilable carbon source or under exclusion of the nitrogen source. A temperature between 0 and 45°C and a pH between 3.5 and 9 is maintained during this stage. Preferably the micro-organisms are kept at a temperature between 20 and 37°C and a pH between 5 and 8. The aerobic conditions required during this stage can be provided according to the abovementioned procedures, provided that the supply of oxygen is sufficient to meet the metabolic requirement of the micro-organisms but also to convert compound (III) into compound (II). The compound (II) produced by the micro-organisms as mentioned above, can be recovered and purified according to any of the well established procedures.

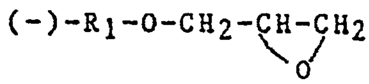 , 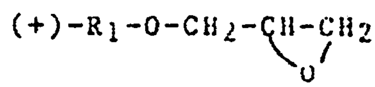

or mixtures thereof produced according to the present invention can be converted into (+)-$R_1$-O-$CH_2$-CHOH-$CH_2$-NH-$R_2$, (-)-$R_1$-O-$CH_2$-CHOH-$CH_2$-NH-$R_2$, or mixtures thereof, respectively by the chemical reaction with isopropylamine or tertiary-butylamine. An example of the reaction to form beta-adrenergic receptor blocking agents with the absolute (S)-configuration from aryl glycidyl ethers with the absolute (S)-configuration is described in the German patent application 2453324.

Especially mixtures with a predominant amount of the compound (-)-$R_1$-O-$CH_2$-CHOH-$CH_2$-NH-$R_2$ can be advantageously used in pharmaceutical products. Preferably those compounds having at least 80% and more preferably at least 90% by weight the S configuration can be used in pharmaceutical products. Compounds of the formula $R_1$–O–$CH_2$–CHOH–$CH_2$–NH–$R_2$ include compounds having a beta-andrenergic receptor blocking property, for example Practolol, Atenolol, Pamatol, Talinolol, H 87/07 and Betaxolol or a pharmaceutical acceptable salt thereof, like an acid addition salt. Examples for pharmaceutically acceptable salts are: Metoprolol tartrate prepared from Metoprolol and L-tartaric acid and 1-isopropylamino-3-phenoxy-2-propanol-hydrochloride prepared from 1-isopropylamino-3-phenoxy-2-propanol and hydrochloric acid. Preferably those beta-adrenergic receptor blocking compounds are produced according to the present inventionwhich are formed to at least 80% and more preferably at least 90& in the S configuration.

The optical purity as will be mentioned in the specification is represented as percent enantiomeric excess: S–R/S+R.

The present invention will be further described with reference to Examples in conjunction with the accompanying Figures, without restricting the scope of the present invention to these Examples.

Brief description of the Figures.

Figure 1 shows the partial pmr spectra of (+) and (±)-4-(2-methoxyethyl)-phenylglycidyl ether in the presence of europium shift reagent

A: (±) or (+)-4-(2-methoxyethyl)-phenylglycidyl ether without shift reagent

B: (±)-4-(2-methoxyethyl)-phenylglycidyl ether with $Eu(hfc)_3$;

Ratio $Eu(hfc)_3$ / (±)-4-(2-methoxyethyl)-phenylglycidyl ether = 0.10

C: (+)-4-(2-methoxyethyl)-phenylglycidyl ether with $Eu(hfc)_3$;

Ratio: $Eu(hfc)_3$ / (+)-4-(2-methoxyethyl)-phenylglycidyl ether = 0.12

Figure 2 shows the pmr spectrum of (-)-metoprolol.

Figure 3 shows the mass spectrum determined by C.I. ($CH_4$) of (-)-metoprolol.

Figure 4 shows the batch production of phenylglycidyl ether by Mycobacterium WMI in a two-phase system (2.25 litre scale) (750 ml cells (6.9 g/l dry wt) + 1500 ml iso-octane containing 2.5% phenylallyl ether).

Figure 5 shows the pmr spectrum of (-)-1-isopropylamino-3-phenoxy-2-propanol.

Figure 6 shows the C.I. ($CH_4$) spectrum of (-)-1-isopropyl-amino-3-phenoxy-2-propanol.

EXAMPLE 1

Transformation of 4-(2-methoxyethyl)-phenylallyl ether into (+)-4-(2-methoxyethyl)-phenylglycidyl ether by Rhodococcus equi NCIB 12035

Approximately half the biomass from a 72 h culture of Rhodococcus equi NCIB 12035, grown at 30°C on tetradecane (1 ml) in ASM mineral salts medium (100 ml), containing yeast extract (0.02%) was transferred to a conical flask (250 ml capacity) containing ASM (50 ml), yeast extract (to 0.02%), tetradecane (0.15 ml), octane (1 ml), Tween 80 (0.05 ml) and 4-(2-methoxyethyl)-phenylallyl ether (0.05 ml). The contents were incubated at 30°C on an orbital shaker and samples were extracted into methylene chloride prior to analysis by gas chromatography on a Varian 3700. (Column: 3%OVI on WHP 100-120, 50 cm x 2 mm i.d., 100°C-200°C at 10°C/min, $N_2$ at 30 cc/min). ASM contains $NH_4Cl$ (0.535 g/l), $KH_2PO_4$ (0.531 g/l), $Na_2HPO_4$ (0.866 g/l), $K_2SO_4$ (0.174 g/l), $MgSO_4.7H_2O$ (0.037 g/l), $CaCl_2.2H_2O$ (0.00735 g/l), $TK_3$ trace elements (1.0 ml/l), and $FeSO_4.7H_2O$ (1.0 ml of a 0.1 M solution/l). $TK_3$ contains $ZnSO_4.7H_2O$ (0.288 g/l), $MnSO_4.4H_2O$ (0.224 g/l), $H_3BO_3$ (0.0618 g/l), $CuSO_4.5H_2O$ (0.1248 g/l), $Na_2MoO_4.2H_2O$ (0.0484 g/l), $CoCl_2.6H_2O$ (0.0476 g/l), KI (0.083 g/l), 1 M $H_2SO_4$ (1 ml/l) at pH 7.0.

The product, (+)-4-(2-methoxyethyl)-phenylglycidyl ether, appeared after 48h incubation and increased until 96h when the level of epoxide was approximately 15% of the remaining 4-(2-methoxyethyl)-phenylallyl ether.

In order to accumulate sufficient quantities of (+)-4-(2-methoxyethyl)-phenylglycidyl ether, the culture broth from 10 x 50 ml and 5 x 500 ml incubations (conditions as described above) was extracted with methylene chloride (350 ml). The extract was dried over $Na_2SO_4$, the solvent evaporated, and the epoxide purified on silica gel using a hexane/ether gradient (epoxide eluted with 30% ether) to give (+)-4-(2-methoxyethyl)-phenylglycidyl ether (353 mg) having $|\alpha|_D^{25} = +8.11°$ (c = 0.95, ethanol).

Optical purity of (+)-4-(2-methoxyethyl)-phenylglycidyl ether was investigated using pmr in the presence of europium shift reagent $Eu(hfc)_3$ (see Figure 1). On addition of the shift reagent, the envelope of signals from each of the geminal protons (labelled (a) and (b)) in the chemically synthesised (±)-4-(2-methoxyethyl)-phenylglycidyl ether (Figure 1A) shifted downfield and each was split into two envelopes of signals of equal intensity (Figure 1B, (a) + (b)). However, under the same conditions, only one envelope of signals was detectable from each of the geminal protons in the microbially produced (+)-4-(2-methoxyethyl)-phenylglycidyl ether (Figure 1C, (a) + (b)). Thus, the optical purity of the (+)-4-(2-methoxyethyl)-phenylglycidyl ether obtained by microbial epoxidation was determined to be 100%, within the experimental error of the pmr measurements.

EXAMPLE II

Conversion of (+)-4-(2-methoxyethyl)-phenylglycidyl ether into (-)-metoprolol.

A solution of (+)-4-(2-methoxyethyl)-phenylglycidyl ether (290 mg. see example I) in dried ethanol (6.2 ml) containing isopropylamine (1.84 ml) was heated under reflux for 3 hours after which the solvent were removed by evaporation. The resulting oil was dissolved in methylene chloride (10 ml) and extracted into 0.2N HCl (10ml) which was then washed with methylene chloride (2 x 10 ml). The acid layer was made basic with 2N NaOH (3 ml) and the product extracted into methylene chloride (10 ml). The methylene chloride extract was dried over $Na_2SO_4$ and the solvent evaporated to give a sticky solid. The product was recrystallized from hexane to give (-)-metopropol (260 mg), showing $|\alpha|_D^{25} = -5.46°$ (c= 1.01, ethanol) and m.p. 42-45°C. (±)-Metoprolol m.p. 49-51°C, prepared chemically, was, as expected optically inactive.

The pmr and mass spectra of (-)-metoprolol (Figures 2-3) and (±)-metoprolol, (not shown), were consistent with the required structure, and were indistinguishable from the pmr spectrum of (±)-metoprolol (m.p. 48.5-50.5°C) extracted from a commercial sample of metoprolol tartrate.

Optical purity of the (-)-metoprolol was determined by separation of its S-leucyl amida diastereomeric amide derivatives on a reverse phase HPLC column (Lichrosorb RP8 (10μm), 25 cm x 1/4" o.d., 4.9 mm i.d., mobile phase: 40% acetonitrile in 0.1 M Na phosphate buffer pH 3.0 at 2.5 ml/min., U.V. detection). Derivatization was achieved by reacting metoprolol with the symmetrical anhydride of tertiary-butoxycarbonyl-S-leucine (BOC-S-leucine) followed by removal of the BOC group with trifluoracetic acid, as described by J. Hermansson and C.J. Von Bahr in J. Chrom., 227, 113 (1982). Analysis of a sample derived from the (±)-metoprolol resulted in two peaks of equal intensity, as expected for a racemate. The sample derived from the (-)-metoprolol gave two peaks with intensity ratio 97.7 : 2.3, equivalent to an optical purity of 95.4%.

EXAMPLE III

Transformation of 4-(2-methoxyethyl)-phenylallyl ether into (+)-4-(2-methoxyethyl)-phenyl glycidyl ether by Pseudomonas aeruginosa NCIB 12036 followed by its transformation into (-)-metoprolol

A suspension of Pseudomonas aeruginosa NCIB 12036 was prepared by resuspending cells (which had been grown in an ASM mineral salts medium containing 0.75% Na lactate and 0.05% diethoxymethane for 24 h at 30°C) in ASM to a volume equal to one tenth of the original culture volume.

Cell suspension (1100 ml) was incubated with 4-(2-methoxyethyl)-phenylallyl ether (3.3 g) at 30°C., 220 rpm. After 24 h, the reaction mixture was extracted with methylene chloride (500 ml), the extract dried over $Na_2SO_4$, and the solvent evaporated to give an oil (2.67 g). Purification on silica gel (as in Example I) gave (+)-4-(2-methoxyethyl)-phenylglycidyl ether (930 mg) with $|\alpha|_D^{25}$ = +8.21° (c = 0.943, ethanol).

Optical purity, as measured by pmr in the presence of europium shift reagent $Eu(hfc)_3$ (as in Example I), was determined to be 100% within the experimental error of the pmr measurements.

(+)-4-(2-methoxyethyl)-phenylglycidyl ether (694 mg) was used (method as in Example II) to prepare (-)-metoprolol (579 mg) m.p. 44-46°C with $|\alpha|_D^{25}$ = -5.49° (c = 1.02, ethanol). Optical purity was determined to be 98% (method as in Example II). The mother liquor was crystallised giving (-)-metoprolol (113 mg) with an optical purity of 96%.

EXAMPLE IV

Transformation of 4-(2-methoxyethyl)-phenylallyl ether into (+)-4-(2-methoxyethyl)-phenyl glycidyl ether by Pseudomonas aeruginosa NCIB 8704 followed by its transformation into (-)-metoprolol

Cell suspension (200 ml) of Pseudomonas aeruginosa NCIB 8704, (preparation as described in Example III) was incubated with 4-(2-methoxyethyl)-phenylallyl ether (2 g) at 30°C for 24 h after which the suspension was extracted and purified (as in Example III) to give (+)-4-(2-methoxyethyl)-phenylglycidyl ether (91 mg). Optical purity of the epoxide, measured by pmr in the presence of europium shift reagent $Eu(hfc)_3$ (as in Example I) was determined to be 100% within the errors of the pmr measurements.

(+)-4-(2-methoxyethyl)-phenylglycidyl ether (76 mg) was used (method as in Example II) to prepare (-)-metoprolol (55 mg), m.p. 42-45°C, $|\alpha|_D^{25}$ = -4.93° (c = 0.944, ethanol), with an optical purity of 98.8% as determined by HPLC of its S-leucyl amide diastereomeric derivatives (as in Example II). Evaporation of the mother liquor yielded (-)-metoprolol (17 mg) of 95.2% optical purity.

EXAMPLE V

Transformation of 4-(2-methoxyethyl)-phenylallyl ether into (+)-4-(2-methoxyethyl)-phenylglycidyl ether by Pseudomonas putida NCIB 9571 followed by its transformation into (-)-metoprolol

Cell suspension (200 ml) of Pseudomonas putida NCIB 9571 (preparation as in Example III) was incubated with 4-(2-methoxyethyl)-phenylallyl ether (1 g) at 30°C for 24 hr, after which it was extracted into methylene chloride and purified (as in Example III) to give (+)-4-(2-methoxyethyl)-phenylglycidyl ether (324 mg) with $|\alpha|_D^{25}$ = + 6.42° (c = 0.88, ethanol). Optical purity as measured by pmr in the presence of europium shift reagent Eu(hfc)3 (as in Example I) was 100% within the experimental errors of the pmr measurements.

(+)-4-(2-methoxyethyl)-phenylglycidyl ether (214 mg) was condensed with isopropylamine (as in Example II) to yield (-)-Metoprolol (146 mg) m.p. 44-46°C with $|\alpha|_D^{25}$ = -5.00° (c = 1.01, ethanol). Optical purity (method as in Example II) was determined to be 98%. Evaporation of the mother liquor yielded (-)-metoprolol (9 mg) of 97.5% optical purity.

EXAMPLE VI

Transformation of 4-(2-methoxyethyl)-phenylallyl ether into (+)-4-(2-methoxyethyl)-phenylglycidyl ether by Pseudomonas oleovorans ATCC 29347 followed by its transformation into (-)-metoprolol

Cell suspension (200 ml) of Pseudomonas oleovorans ATCC 29347 (preparation as in Example III) was incubated with 4-(2-methoxyethyl)-phenylallyl ether (2 g) at 30°C for 5 h. Extraction into methylene choride and purification on silica gel (as in Example I) yielded (+)-4-(2-methoxyethyl)-phenylglycidyl ether (289 mg) with $|\alpha|_D^{25}$ = +8.02° (c = 1.16, ethanol). Optical purity, as measured by pmr in the presence of europium shift reagent $Eu(hfc)_3$ (as in Example I) was determined to be 100% within the experimental errors of the pmr measurements.

(+)-4-(2-methoxyethyl)-phenylglycidyl ether (171 mg) was reacted with isopropylamine (method as in Example II) to give (-)-metoprolol (154 mg) m.p. 44-45°C with $|\alpha|_D^{25}$ = -5.27° (c = 0.967, ethanol). Optical

purity was determined to be 98.4% based of HPLC of the S-leucyl amide diastereomeric derivatives (as in Example II). Evaporation of the mother liquor gave (-)-metoprolol (6 mg) of 92.2% optical purity.

EXAMPLE VII

Microbial transformation of 4-(2-methoxyethyl)-phenylallyl ether into (+)-4-(2-methoxyethyl)-phenylglycidyl ether

Mineral salts medium (50 ml) containing Tween 80 (0.05 ml), tetradecane (0.05 ml) and 4-(2-methoxyethyl)-phenylallyl ether (0.05 ml) was inoculated with either Nocardia corallina ATCC 31338, Rhodococcus sp. NCIB 11277 or Mycobacterium rhodochrous NCIB 9703 (all pregrown for 72 h on 0.5% tetradecane), then incubated at 30°C. Samples were taken at 24 h and 96 h, extracted into methylene chloride and analysed by gas chromatography (GC).

In each case, peaks corresponding to 4-(2-methoxyethyl)-phenylglycidyl ether were detected with conversions as follows: Mycobacterium rhodochrous 2% after 24 h; Rhodococcus sp. 1% after 96 h and Nocardia corallina 5% after 96 h. Further confirmation of the structure was obtained when the contents of a scaled up experiment using Nocardia corallina (500 ml culture, conditions as above) were extracted into methylene chloride followed by purification on silica gel and pmr analysis. The pmr europium shift spectra of the 4-(2-methoxyethyl)-phenylglycidyl ether suggested an optical purity of 100%, and were identical to the europium pmr spectra of (+)-4-(2-methoxyethyl)-phenylglycidyl ether obtained from Rhodococcus equi.

EXAMPLE VIII

Transformation of phenylallyl ether into (+)-phenylglycidyl ether by Mycobacterium NCIB 11626 followed by its transformation into (-)-isopropylamino-3-phenoxy-2-propanol

Continuous culture conditions: culture volume 2.7 litres, medium: AM2 (containing $(NH_4)_2SO_4$ (1.45 g/l), $H_3PO_4$ (1.0 g/l), $MgSO_4.7H_2O$ (0.099 g/l), $CaCl_2.2H_2O$ (0.015 g/l), $TK_3$ trace elements solution (2 ml/l), 0.1 M $FeSO_4.7H_2O$ (1 ml/l)). Carbon source: ethylene at 25 ml/min, air at 300 ml/min, stirrer speed: 280 rpm, temperature 28°C, pH 6.8, dilution rate: 0.02 $h^{-1}$ ($\mu_{max}$ = ca. 0.03 $h^{-1}$). These conditions supported a biomass level of ca. 3.2 g/l dry weight. Cells were generally concentrated before use (by centrifugation followed by resuspension in spent medium).

Phenylglycidyl ether was analysed by GC (column and conditions as in previous Examples except temperature programme was 50°C-100°C at 10°C/min).

A 2.25 litre batch two-phase system was used to produce phenylglycidyl ether. The transformation was carried out in a fermenter containing 750 ml cell suspension (6.9 g/l dry weight) and 1500 ml iso-octane containing 2.5% v/v phenylallyl ether (Figure 4). The epoxide was produced at a rate of 193 µmol/h/g dry weight (0.03 g/h/g dry weight) and was isolated after 10 h by chromatography on silica gel then purified by distillation to give (+)-phenylglycidyl ether (750 mg).

The pmr spectra and mass spectra of (+)-phenylglycidyl ether produced microbially were consistent with the required structure and identical to those of (±)-phenylglycidyl ether prepared chemically by the reaction of phenylallyl ether with m-chloroperbenzoic acid. The (+)-phenylglycidyl ether gave a specific rotation of $|\alpha|_D^{25}$ = +11.38° (c = 1.09, ethanol).

Optical purity was investigated using pmr in the presence of europium shift reagent $Eu(hfc)_3$, the same as in Example I. On addition of the shift reagent, the envelope of signals from each of the geminal protons in the chemically synthesised (±)-phenylglycidyl ether shifted downfield and were each split into two envelopes of signals of equal intensity, whereas the envelope of signals from the geminal protons in microbially produced (+)-phenylglycidyl ether were split in the ratio 6.7 : 1. This result indicates that 87% of the molecules are in one enantiomeric form, which implies an optical purity of 74%. Analysis of a mixture of epoxides from microbial and chemical sources gave signal ratios which confirmed that 90% of the (+)-phenylglycidyl ether was in one enantiomeric form, equivalent to an optical purity of 80%.

Further confirmation of the optical purity of the (+)-phenylglycidyl ether was established following its conversion into (-)-1-isopropylamino-3-phenoxy-2-propanol. (+)-phenylglycidyl ether (300 mg) was reacted with isopropylamine (as in Example II) to give (-)-1-isopropylamino-3-phenoxy-2-propanol (125 mg) with $|\alpha|_D^{25}$ = -6.51° (c = 0.984, ethanol), m.p. 42-44°C.

(±)-1-Isopropylamino-3-phenoxy-2-propanol was similarly synthesised from (±)-phenylglycidyl ether. The pmr and mass spectra of (-)-1-isopropylamino-3-phenoxy-2-propanol (Figures 5-6) and (±)-1-isopropylamino-3-phenoxy-2-propanol (not shown) were consistent with the required structure. Optical purity was measured by HPLC of the corresponding S-leucyl amide diastereomeric derivatives (as in Example II) and determined to be 80%.

EXAMPLE IX

Transformation of 4-allyloxy-phenylacetamide into (+)-4-(2,3-epoxypropyloxy)-phenylacetamide by Pseudomonas oleovorans ATCC 29347 followed by its transformation into (-)-1-(p-carbamoylmethylphenoxy-3-isopropylamino-2-propanol ( (-)- Atenonol)

A suspension of Pseudomonas oleovorans ATCC 29347 was prepared by resuspending cells (which had been grown in PSX mineral salts medium, pH 7, containing 0.75% sodium lactate and 0.05 % diethoxymethane for 24 hours at 30°C) in PSX, pH 7.5, to a volume equal to one tenth of the original culture volume. PSX contains: $KH_2PO_4$ (8.92 g/l), $Na_2HPO_4$ (2.94 g/l), $(NH_4)_2$ $HPO_4$ (1.0 g/l), $(NH_4)_2SO_4$ (0.2 g/l) KCl (0.2 g/l), $Na_3$citrate (0.294 g/l), $CaSO_4.2H_2O$ (0.005 g.l), $MgSO_4.7H_2O$ (0.2 g/l), and PS II trace elements solution (10 ml/l). (PS II trace elements solution contains: $(NH_4)_2SO_4.FeSO_4.6H_2O$ (0.25 g/l), $ZnSO_4.7H_2O$ (0.05 g/l), $MnCl_2.4H_2O$ (0.03 g/l), $CuSO_4.5H_2O$ (0.015 g/l), $CoCl_2.6H_2O$ (0.015 g/l), $H_3BO_3$ (0.005 g/l), $Na_2MoO_4.2H_2O$ (0.0055 g/l), KI (0.01 g/l), HCl to pH 3).

Cell suspension (1100 ml) was incubated with 4-allyloxy-phenylacetamide (3.2 g) added as a 10% ball-milled suspension in water (containing 5% Tween 80), in eleven 2.5 litre conical flasks on an orbital shaker at 30°C. After 72 hours, the incubation mixture was extracted with methylene chloride (1000 ml), the solvent was dried and evaporated, after which the residue was recrystallized from acetone to give 2.27 g white crystalline material whose composition was 90% 4-allyloxy-phenylacetamide and 10% 4-(2,3-epoxypropyloxy)-phenylacetamide as determined by HPLC (column: Lichrosorb RP8 (10 µm) 25 cm x 1/4"o.d., 4.9 mm i.d., mobile phase 40% acetonitrile in water at 2 ml/min, UV. detection).

The crystallized extract, containing approximately 230 mg 4-(2,3-epoxy-propyloxy)-phenylacetamide, was dissolved in dry ethanol (40 ml), then heated under reflux with isopropylamine (2.5 ml) for 5 hours, after which the excess solvent was evaporated. The residue was dissolved in methylene chloride (50 ml) and extracted with 3 x 50 ml 0.2N HCl. The acid layers were combined, washed with methylene chloride, then made basic with 5N NaOH. The white precipitate which was formed, was extracted into methylene chloride (3 x 100 ml), the extract was dried over $Na_2SO_4$, then the solvent was evaporated to give a white solid which was recrystallized from hexane/ethyl acetate giving (-)-Atenolol (76 mg) $|\alpha|_D^{25}$ = -3.9° (c=1. 01, EtOH), m.p. 146°-148°C, $^1H$ NMR ($CHCl_3$) 360 MHz spectrum : delta 1.1 (d, 6H, <u>$CH_3$</u>), delta 1.5-2.5 broad (s, 2H, <u>NH</u>, <u>OH</u>), delta 2.82 (m, 1H, NH<u>CH</u>), delta 2.75/2.95 (m, 2H, <u>$CH_2$</u>NH), delta 3.53 (s, 2H, CO-<u>$CH_2$</u>), delta 3.98 (m, 3H, -O-<u>$CH_2$</u>-<u>CH</u>-), delta 5.35 broad (s, 2H, <u>$NH_2$</u>), delta 6.9 (d, 2H, para substituted aromatic), delta 7.2 (d, 2H, para substituted aromatic). Chemical ionization ($NH_3$) mass spectrum gave a molecular ion $(M+H)^+$ m/e 267, indicating a molecular weight of 266, consistent with the expected mass. The pmr and mass spectra were identical to those of (±)-Atenolol prepared chemically.

Optical purity of the (-)-Atenolol was determined by separation of its di-benzoyl-tartaric acid monoester diastereomeric derivatives on a reverse phase HPLC column (Lichrosorb RP8 (10 µm), 25 cm x 1/4" o.d., 4.9 mm i.d., mobile phase 2% acetic acid (to pH 3.7 with $NH_3$) 50 : 50 methanol at 1.5 ml/min.). Derivatization was achieved by reacting (±)- or (-)-Atenolol with (R,R)-O,O-dibenzoyltartaric anhydride as described by W. Linder et al in J. Chrom. 316, p. 605-616 (1984). Analysis of a sample derived from (±)-Atenolol resulted in the formation of two diastereomer peaks of near equal intensity, whereas the sample derived from the (-)-Atenolol resulted in the formation of two peaks with intensity ratio 1.5 : 98.5, equivalent to an optical purity of 97.0%.

EXAMPLE X

Transformation of 4-allyloxy-phenylacetamide into 4-(2,3-epoxypropyloxy)-phenylacetamide by Pseudomonas oleovorans ATCC 29347

A suspension of Pseudomonas oleovorans ATCC 29347 was prepared by resuspending cells (which had been grown for 24 hours at 30°C in PSX mineral salts medium, pH 7, containing 0.75 % glycerol plus 0.05% diethoxymethane) in PSX, pH 7.5, to a volume equal to one tenth of the original culture volume.

Cell suspension (10 ml, 12.6 g/l dry wt.) was incubated at 30°C with 0.5 ml of a 90 mg/ml ball-milled and sonicated suspension of 4-allyloxy-phenyl-acetamide (which also contained 50 mg/ml Tween 80 ) in a 250 ml stoppered conical flask on an orbital incubator. Samples (1 ml) were extracted into methylene chloride (2 ml), the solvent was evaporated, then the residue was redissolved in dimethylformamide prior to analysis by HPLC (conditions as for analysis of 4-(2,3-epoxypropyloxy)-phenylacetamide in example IX). The level of 4-(2,3-epoxypropyloxy)-phenyl-acetamide in the incubation mixture reached 0.9 g/l and 1.3 g/l after 3 hours and 24 hours respectively.

Example XI

Transformation of 4-allyloxy-phenylacetamide into 4-(2,3-epoxypropyloxy)-phenylacetamide by Pseudomonas aeruginosa NCIB 12036, Pseudomonas aeruginosa NCIB 8704 and Pseudomonas putida NCIB 9571.

Cell suspensions of Pseudomonas aeruginosa NCIB 12036, Pseudomonas aeruginosa NCIB 8704 and Pseudomonas putida NCIB 9571 were prepared by resuspending cells (which had been grown in PSX mineral salts medium, pH 7, containing 0.75 % sodium lactate and 0.05 % diethoxymethane for 24 hours at 30°C) in PSX, pH 7.5, to a volume equal to one tenth of the original culture volume.

Cell suspension (10 ml) was incubated with 0.5 ml of a 10% ball-milled aqueous suspension of 4-allyloxyphenylacetamide (which also contained 5%Tween 80) at 30°C in stoppered 250 ml conical flasks on an orbital incubator. After 24 hours the incubation mixtures were extracted into methylene chloride and found to contain 4-(2,3-epoxypropyloxy)-phenyl-acetamide at concentrations as follows: Pseudomonas aeruginosa NCIB 12036, 0.07 g/l; Pseudomonas aeruginosa NCIB 8704, 0.08 g/l; Pseudomonas putida NCIB 9571, 0.19 g/l (analysis by HPLC as in previous examples).

Example XII

Transformation of 4-(2-methoxyethyl)-phenylallyl ether into 4-(2-methoxyethyl)-phenylglycidyl ether by Pseudomonas oleovorans ATCC 29347.

A suspension of Pseudomonas oleovorans ATCC 29347 was prepared by resuspending cells (which had been grown to stationary phase in PSX mineral salts medium, pH 7, containing 0.75 % glycerol and 0.05 % diethoxymethane, at 30°C) in PSX, pH 7.5, to a volume equal to one tenth of the original culture volume.

Cell suspension (10 ml, dry wt. 12.6 g/l, contained in a 250 ml conical flask) was incubated with 4-(2-methoxyethyl)-phenylallyl ether (250 µl) and glucose (50 mg) at 37°C on an orbital shaker. Formation of 4-(2-methoxyethyl)-phenylglycidyl ether was monitored by gas chromatography after extraction into methylene chloride (conditions as described in previous examples). The level of 4-(2-methoxyethyl)-phenylglycidyl ether in the incubation mixture was 7.30 g/l after six hours.

## Claims

1. A process for the preparation of a pharmaceutically active compound in a stereospecific form of the formula

$$R_1\text{–O–}CH_2\text{–CHOH–}CH_2\text{–NH–}R_2 \quad (I)$$

or a pharmaceutically acceptable salt thereof, like an acid addition salt, and/or a compound in a stereospecific form of the formula

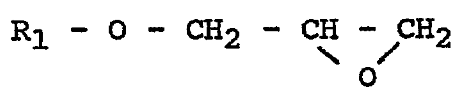 (II) ,

wherein $R_1$ is an optionally substituted phenyl group and wherein $R_2$ is an alkyl group of 2 to 6 carbon atoms, this alkyl group being optionally substituted, which comprises subjecting a compound of the formula

$$R_1\text{–O–}CH_2\text{–CH=}CH_2 \quad (III)$$

to the action of a bacterium belonging to the genus Rhodococcus, the genus Mycobacterium, the genus Nocardia or the genus Pseudomonas, having the ability in a suitable medium for stereoselective epoxidation of compound (III) into compound (II), whereby at least 90% by weight of compound (II) is formed in the S configuration, at least partly separating compound (II) and/or reacting compound (II) with a $C_2$–$C_6$ alkylamine the alkyl group of which is optionally substituted and at least partly separating compound (I) and/or converting compound (I) into the pharmaceutically acceptable salt thereof.

2. A process according to claim 1, wherein isopropylamine or tertiary-butylamine is used.

3. A process according to claim 1 or 2 wherein $R_1$ is a 4-substituted phenyl group.

4. A process according to any one of claims 1–3 wherein $R_1$ is a 4-acetamidophenyl, 4-carbamoylmethylphenyl, 4-[2-(methylformylamino)-ethyl]phenyl, phenyl,
4-((3-cyclohexylureido)-phenyl,
4-(2-methoxyethoxy)-phenyl, or
4-[2-(cyclopropylmethoxy)-ethyl]-phenyl group.

5. A process according to any one of claims 1–3 wherein $R_1$ is a 4-carbamoylmethylphenyl group.

6. A process according to any one of claims 1–3 wherein $R_1$ is a phenyl group.

7. A process according to claim 1, wherein said bacterium is of the genus Rhodococcus.

8. A process according to claim 1, wherein said bacterium is of the genus Mycobacterium.

9. A process according to claim 1, wherein said bacterium is of the genus Nocardia.

10. A process according to claim 1, wherein said bacterium is of the genus Pseudomonas.

11. A process according to any one of the preceding claims wherein said bacterium is immobilized with polymer gel.

12. A process as claimed in claim 1, wherein $R_1$ is a phenyl group and $R_2$ is an isopropyl group and the bacterium used is Mycobacterium WMI, preferably Mycobacterium WMI NCIB 11626.

13. A process as claimed in claim 1, wherein $R_1$ is a 4-carbamoylmethylphenyl group and $R_2$ is an isopropyl group and the bacterium used is <u>Pseudomonas oleovorans</u>, preferably <u>Pseudomonas oleovorans</u> ATCC 29347.

## Patentansprüche

1. Verfahren zur Herstellung einer pharmazeutisch aktiven Verbindung der Formel:

$$R_1–O–CH_2–CHOH–CH_2–NH–R_2 \text{ (I)}$$

in einer stereospezifischen Form oder eines pharmazeutisch unbedenklichen Salzes davon, wie eines Säureadditionssalzes, und/oder einer Verbindung der Formel :

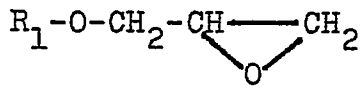

(II)

in einer stereospezifischen Form, wobei $R_1$ eine gegebenenfalls substituierte Phenylgruppe ist und wobei $R_2$ eine Alkylgruppe mit 2 bis 6 Kohlenstoffatomen ist, die gegebenenfalls substituiert ist, dadurch gekennzeichnet, daß man eine Verbindung der Formel:

$$R_1–O–CH_2–CH{=}CH_2 \text{ (III)}$$

der Einwirkung eines Bakteriums unterwirft, das zu der Gattung Rhodococcus, der Gattung Mycobacterium, der Gattung Nocardia oder der Gattung Pseudomonas gehört und das in einem geeigneten Medium die Fähigkeit zur stereoselektiven Epoxydierung von Verbindung (III) zu Verbindung (II) hat, wobei mindestens 90 Gew.-% von Verbindung (II) in der S-Konfiguration gebildet werden, Verbindung (II) mindestens teilsweise abtrennt und/oder Verbindung (II) mit einem Alkylamin mit 2 bis 6 Kohlenstoffatomen umsetzt, dessen Alkylgruppe gegebenenfalls substituiert ist, und Verbindung (I) mindestens teilweise abtrennt und/oder Verbindung (I) in das pharmazeutisch unbedenkliche Salz davon überführt.

2. Verfahren nach Anspruch 1, dadurch gekennzeichnet, daß man Isopropylamin oder tert.-Butylamin verwendet.

3. Verfahren nach Anspruch 1 oder 2, dadurch gekennzeichnet, daß $R_1$ eine in 4-Stellung substituierte Phenylgruppe ist.

4. Verfahren nach einem der Ansprüche 1 bis 3, dadurch gekennzeichnet, daß $R_1$ eine 4-Acetamidophenyl-, 4-Carbamoylmethylphenyl-, 4-[2-(Methylformylamino)-ethyl]-phenyl-, Phenyl-, 4-(3-Cyclohexylureido)-phenyl-, 4-(2-Methoxyethoxy)-phenyl- oder 4-[2-(Cyclopropylmethoxy)-ethyl]-phenylgruppe ist.

5. Verfahren nach einem der Ansprüche 1 bis 3, dadurch gekennzeichnet, daß $R_1$ eine 4-Carbamoylmethylphenylgruppe ist.

6. Verfahren nach einem der Ansprüche 1 bis 3, dadurch gekennzeichnet, daß $R_1$ eine Phenylgruppe ist.

7. Verfahren nach Anspruch 1, dadurch gekennzeichnet, daß das Bakterium zu der Gattung Rhodococcus gehört.

8. Verfahren nach Anspruch 1, dadurch gekennzeichnet, daß das Bakterium zu der Gattung Mycobacterium gehört.

9. Verfahren nach Anspruch 1, dadurch gekennzeichnet, daß das Bakterium zu der Gattung Nocardia gehört.

10. Verfahren nach Anspruch 1, dadurch gekennzeichnet, daß das Bakterium zu der Gattung Pseudomonas gehört.

11. Verfahren nach einem der vorangehenden Ansprüche, dadurch gekennzeichnet, daß das Bakterium mit Polymergel immobilisiert ist.

12. Verfahren nach Anspruch 1, dadurch gekennzeichnet, daß $R_1$ eine Phenylgruppe ist und $R_2$ eine Isopropylgruppe ist und das verwendete Bakterium Mycobacterium WMI, vorzugsweise Mycobacterium WMI NCIB 11626, ist.

13. Verfahren nach Anspruch 1, dadurch gekennzeichnet, daß $R_1$ eine 4-Carbamoylmethylphenylgruppe ist und $R_2$ eine Isopropylgruppe ist und das verwendete Bakterium Pseudomonas oleovorans, vorzugsweise Pseudomonas oleovorans ATCC 29347, ist.

## Revendications

1. Procédé de préparation d'un composé pharmaceutiquement actif sous une forme stéréospécifique de la formule

$$R_1–O–CH_2–CHOH–CH_2–NH–R_2 \quad (I)$$

ou d'un sel pharmaceutiquement acceptable de celui-ci, comme un sel d'addition d'acide, et/ou d'un composé sous une forme stéréospécifique de la formule

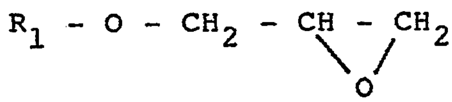

(II)

où $R_1$ est un radical phényle éventuellement substitué et où $R_2$ est un radical alcoyle de 2 à 6 atomes de carbone, ce radical alcoyle étant éventuellement substitué, qui comprend l'exposition d'un composé de formule

$$R_1–O–CH–CH=CH_2 \quad (III)$$

à l'action d'une bactérie appartenant au genre Rhodococcus, au genre Mycobacterium, au genre Nocardia ou au genre Pseudomonas ayant l'aptitude, dans un milieu approprié, à l'époxydation stéréosélective du composé (III) en le composé (II), par laquelle le composé (II) est formé pour au moins 90% en poids dans la configuration S, la séparation au moins partielle du composé (II) et/ou la réaction du composé (II) avec une $C_2$–$C_6$-alcoylamine dont le radical alcoyle est éventuellement substitué, et la séparation au moins partielle du composé (I) et/ou la conversion du composé (I) en le sel pharmaceutiquement acceptable de celui-ci.

2. Procédé suivant la revendication 1, dans lequel l'isopropylamine ou la t-butylamine sont utilisées.

3. Procédé suivant la revendication 1 ou 2, dans lequel $R_1$ est un radical phényle substitué en position 4.

4. Procédé suivant l'une quelconque des revendications 1 à 3, dans lequel $R_1$ est un radical 4-acétamidophényle, 4-carbamoylméthylphényle, 4[2-(méthylformylamino)-éthyl]phényle, phenyle, 4-(3-cyclohexyluréido)-phényle, 4-(2-méthoxyéthoxy)-phényle ou 4-[2-(cyclopropylméthoxy)-éthyl]-phényle.

5. Procédé suivant l'une quelconque des revendications 1 à 4, dans lequel $R_1$ est un radical 4-carbamoylméthylphényle.

6. Procédé suivant l'une quelconque des revendications 1 à 3, dans lequel $R_1$ est un radical phényle.

7. Procédé suivant la revendication 1, dans lequel la bactérie appartient au genre Rhodococcus.

8. Procédé suivant la revendication 1, dans lequel la bactérie appartient au genre Mycobacterium.

9. Procédé suivant la revendication 1, dans lequel la bactérie appartient au genre Nocardia.

10. Procédé suivant la revendication 1, dans lequel la bactérie appartient au genre Pseudomonas.

11. Procédé suivant l'une quelconque des revendications précédentes, dans lequel la bactérie est immobilisée par un gel de polymère.

12. Procédé suivant la revendication 1, dans lequel $R_1$ est un radical phényle et $R_2$ est un radical isopropyle et la bactérie utilisée est Mycobacterium WMI, de préférence Mycobacterium WMI NCIB 11626.

13. Procédé suivant la revendication 1, dans lequel $R_1$ est un radical 4-carbamoylméthylphényle et $R_2$ est un radical isopropyle et la bactérie utilisée est Pseudomonas oleovorans, de préférence Pseudomonas oleovorans ATCC 29347.

FIG. Ia

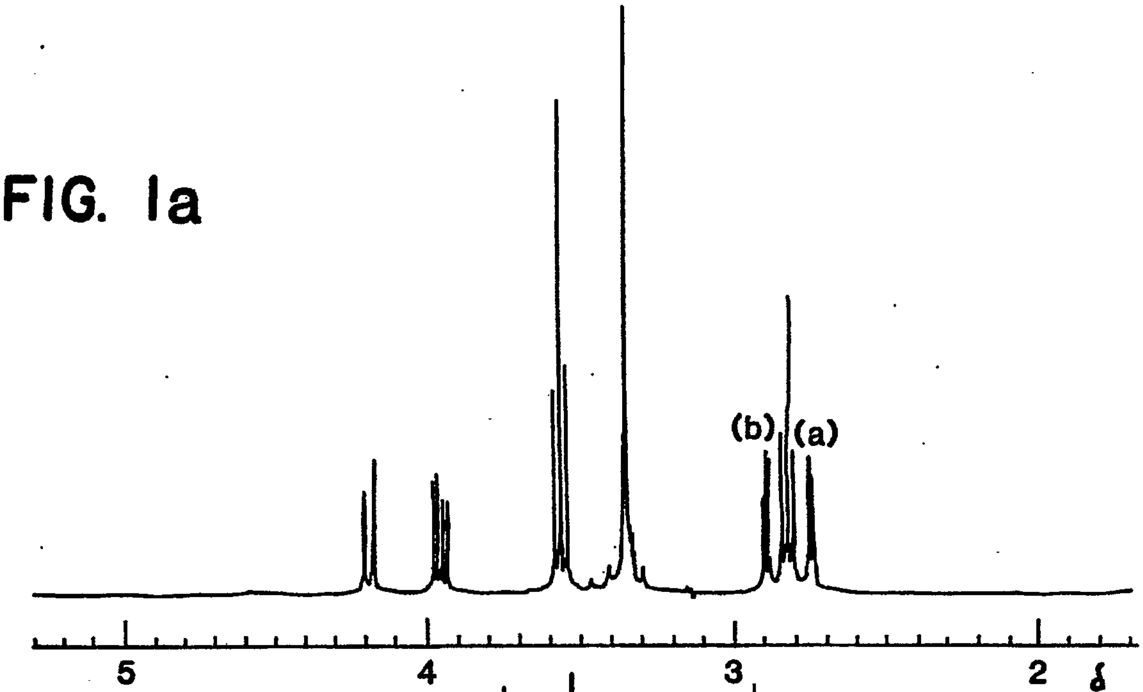


FIG. Ib

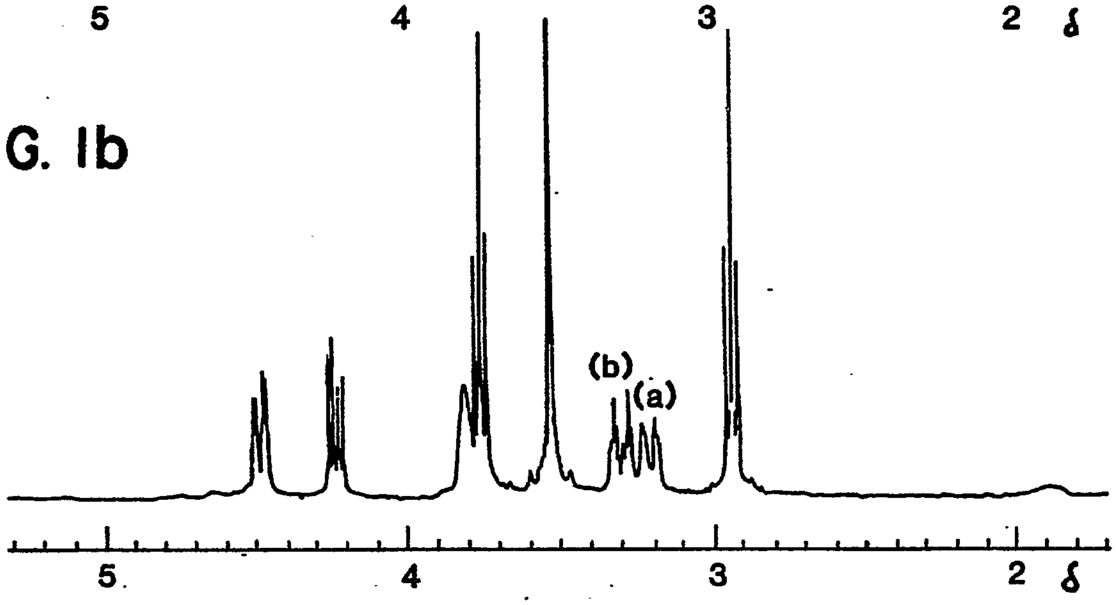


FIG. Ic

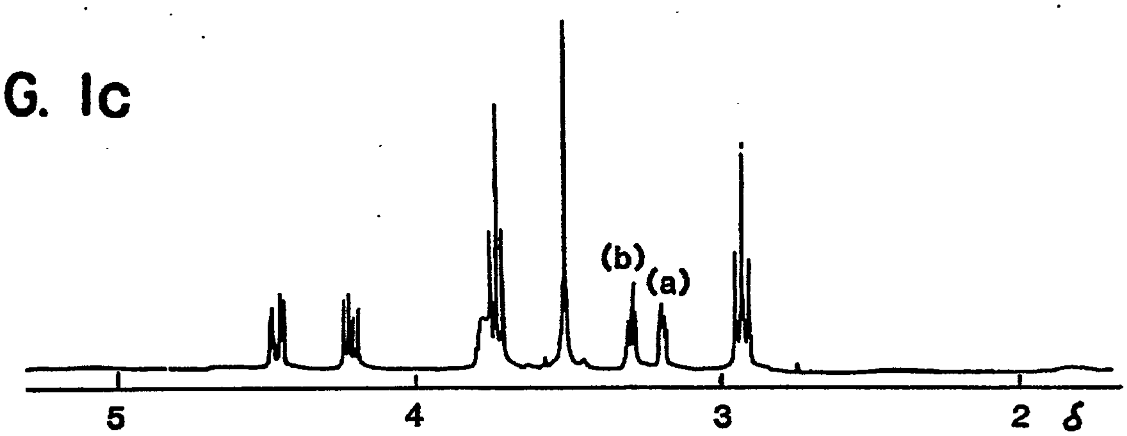

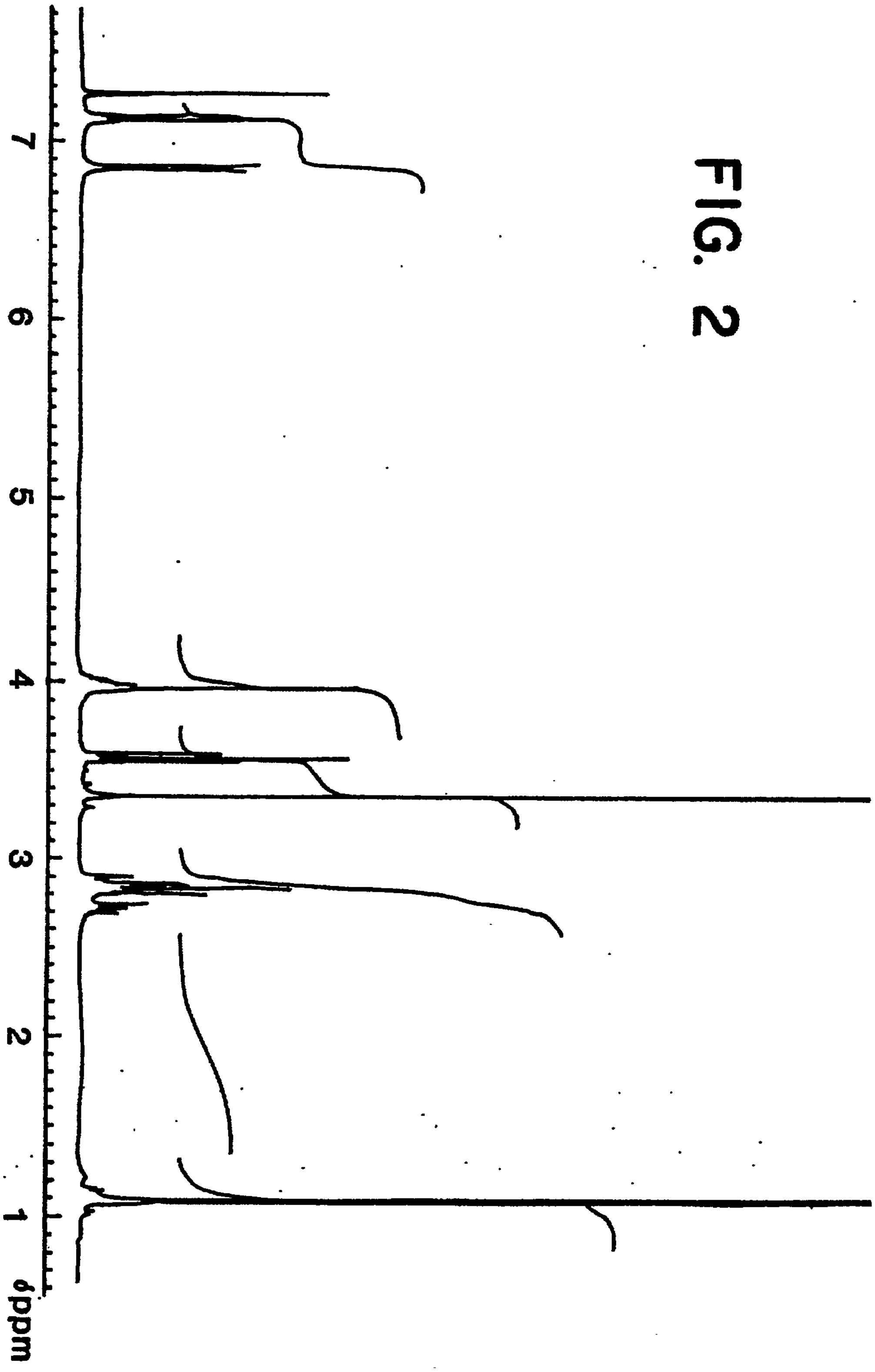


FIG. 2

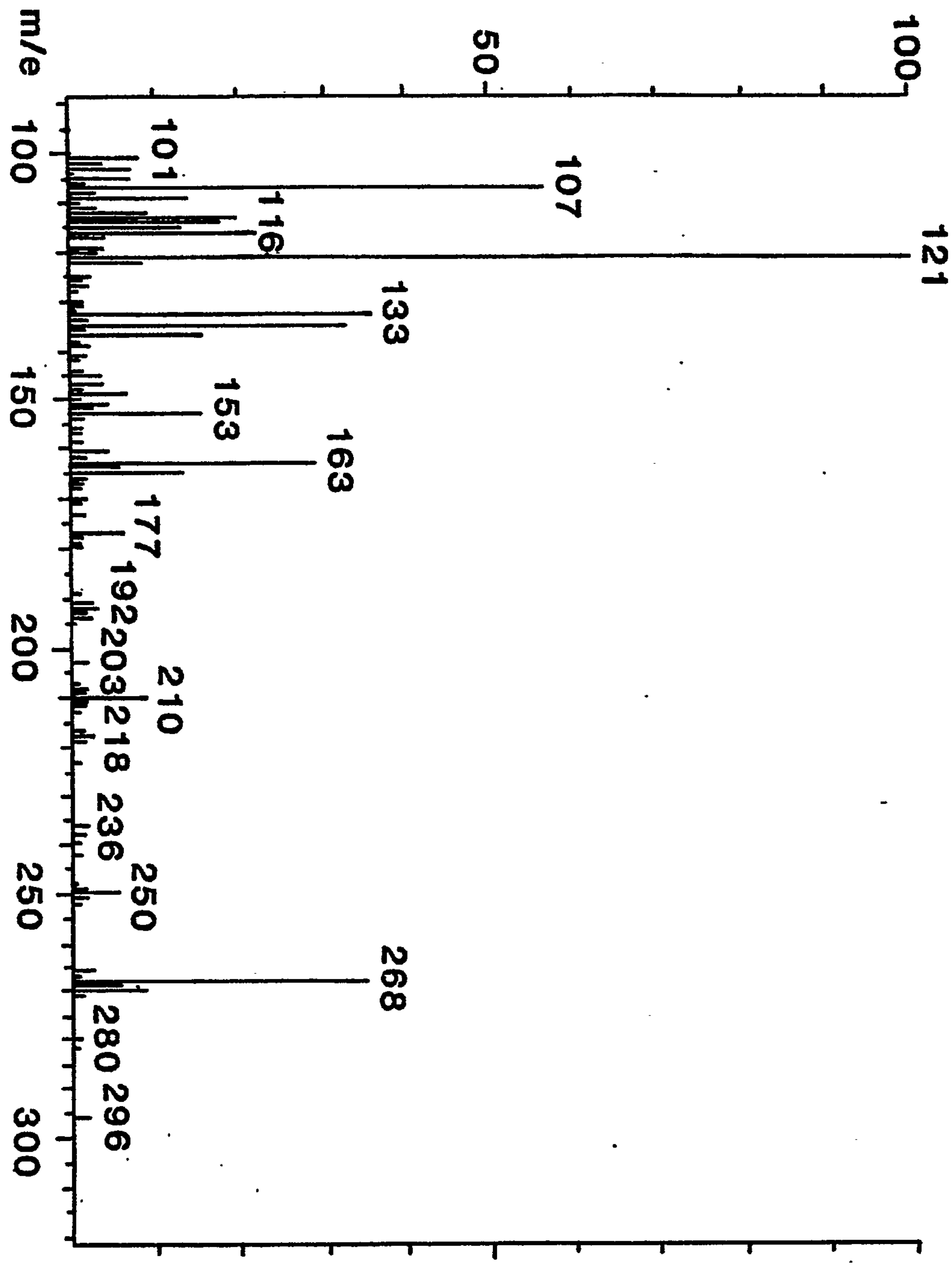
m/e
100
50
100
150
200
250
300
101
107
116
121
133
153
163
177
192
203
210
218
236
250
268
280
296

FIG. 3

FIG. 4

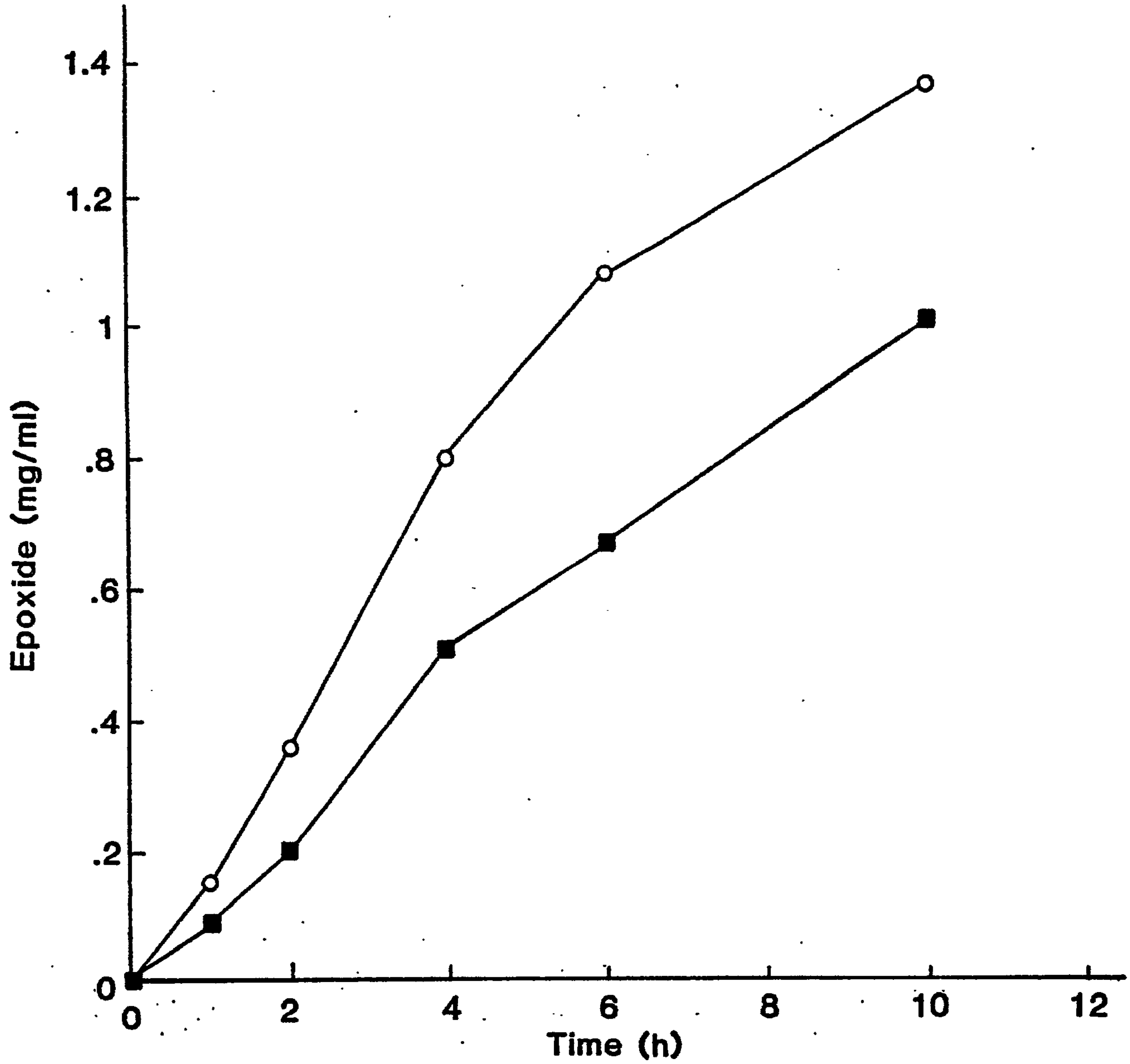


■——— = Level of phenylglycidyl ether in isooctane layer
○——— = level of phenylglycidyl ether in a control shake flask experiment running in parallel (4 ml cells +10 ml isooctane containing 2.5% phenylallyl ether)

FIG. 5

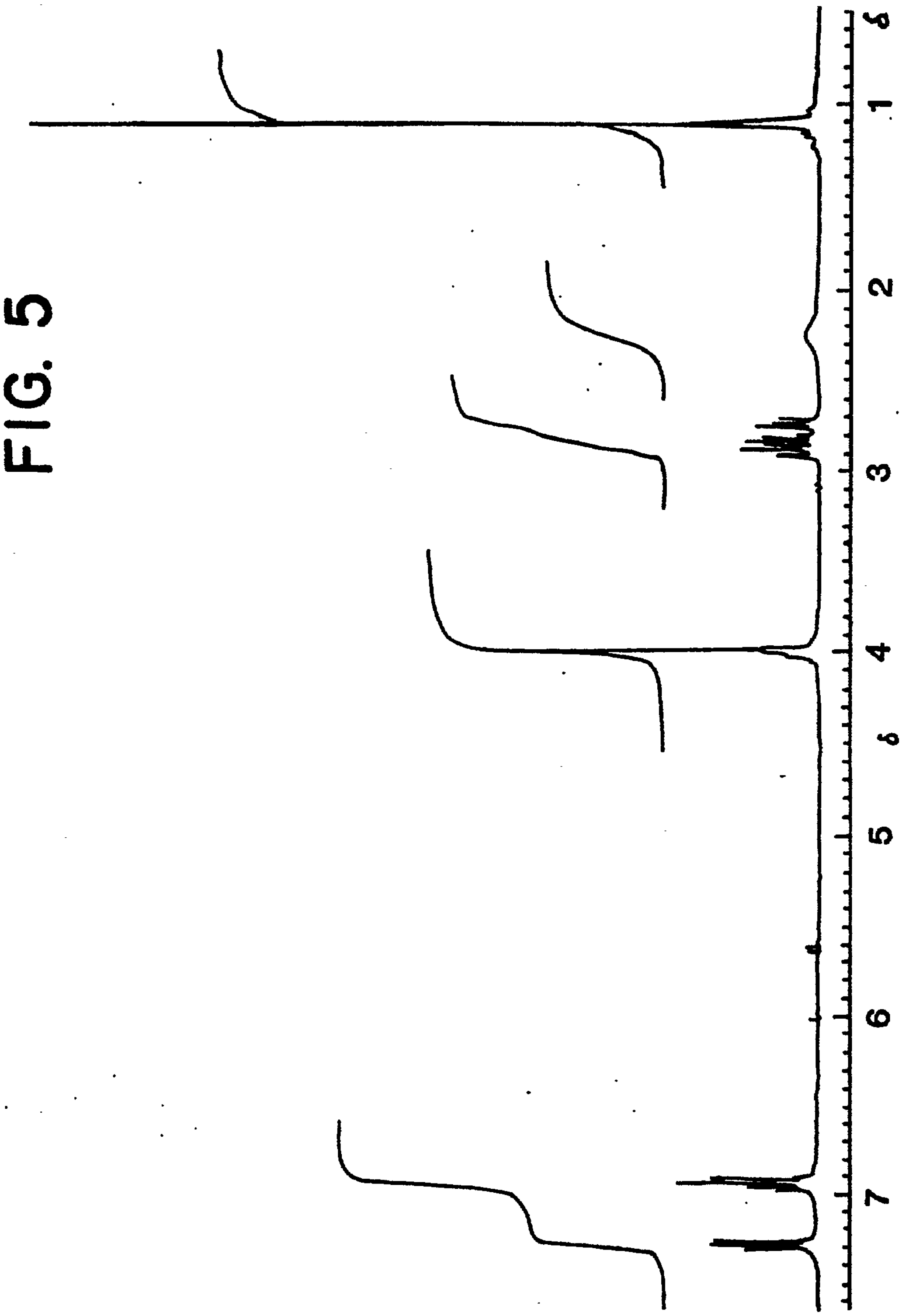

FIG. 6

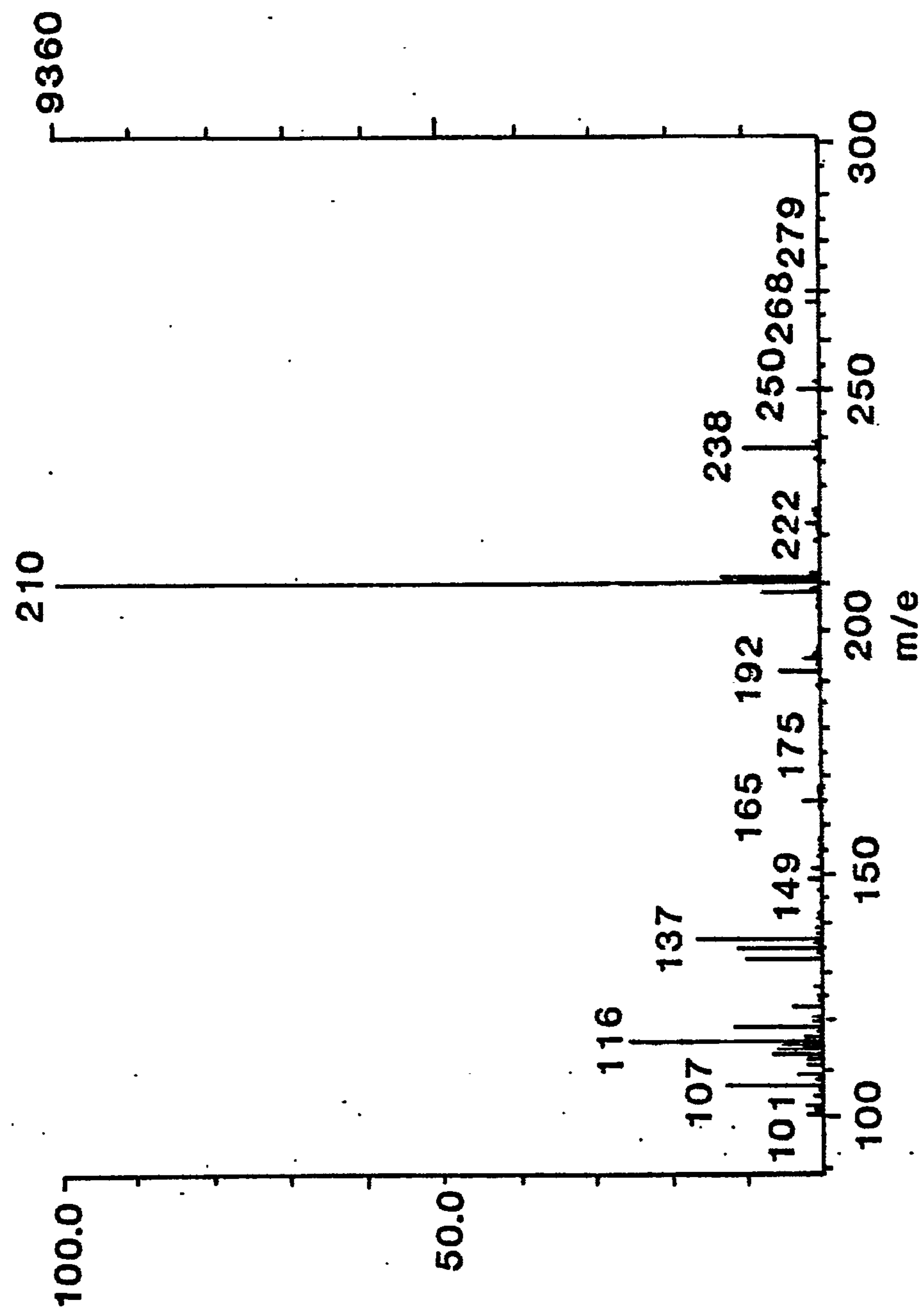
9360
100.0
50.0
100
150
200
250
300
m/e
101
107
116
137
149
165
175
192
210
222
238
250
268
279